(19) Europäisches Patentamt
European Patent Office
Office européen des brevets

(11) Publication number: 0 315 828 B1

(12) # EUROPEAN PATENT SPECIFICATION

(45) Date of publication of patent specification: **08.04.92**

(51) Int. Cl.5: **C07D 513/04**, A61K 31/47

(21) Application number: **88117810.7**

(22) Date of filing: **26.10.88**

The file contains technical information submitted after the application was filed and not included in this specification

(54) **Ouinolinecarboxylic acid derivatives.**

(30) Priority: **07.11.87 JP 281550/87**

(43) Date of publication of application:
**17.05.89 Bulletin 89/20**

(45) Publication of the grant of the patent:
**08.04.92 Bulletin 92/15**

(84) Designated Contracting States:
**AT BE CH DE ES FR GB IT LI NL SE**

(56) References cited:
**EP-A- 0 067 411**
**EP-A- 0 191 390**
**EP-A- 0 315 827**
**ZA-A- 8 703 179**

(73) Proprietor: **NIPPON SHINYAKU COMPANY, LIMITED**
**14, Kisshoin Nishinosho Monguchicho**
**Minami-ku Kyoto-shi Kyoto 601(JP)**

(72) Inventor: **Kise, Masahiro**
**Higashiiru Tominokoji Anayekoji-dori**
**Nakakyo-ku Kyoto 604(JP)**
Inventor: **Kitano, Masahiko**
**9-4, Donouecho Matsugasaki**
**Sakyo-ku Kyoto 606(JP)**
Inventor: **Ozaki, Masakuni**
**7-25 Mitani Terada**
**Joyo City 610-01(JP)**
Inventor: **Kazuno, Kenji**
**151-16 Kohirai**
**Rittocho 520-30(JP)**
Inventor: **Matsuda, Masahito**
**19-16 Seifucho**
**Otsu City 520-02(JP)**
Inventor: **Shirahase, Ichiro**
**Nippon Shinyaku Yamashina Dorm 39**
**Sakanotsujicho**
**Oyake Yamashinaku Kyoto 607(JP)**
Inventor: **Segawa, Jun**
**Daiichi Rakusei Haitsu 405 9 Biwacho**
**Karahashi Minami-ku Kyoto 601(JP)**

(74) Representative: **Hranitzky, Wilhelm Max et al**
**NOVAPAT- CABINET CHEREAU 9, rue du Valais**
**CH-1202 Genève(CH)**

**EP 0 315 828 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention relates to novel quinolinecarboxylic acid derivatives exhibiting antibacterial activity and useful as remedies for various infectious diseases. More particularly, it relates to quinolinecarboxylic acid derivative represented by the following general formula (I) and physiologically-acceptable salt thereof.

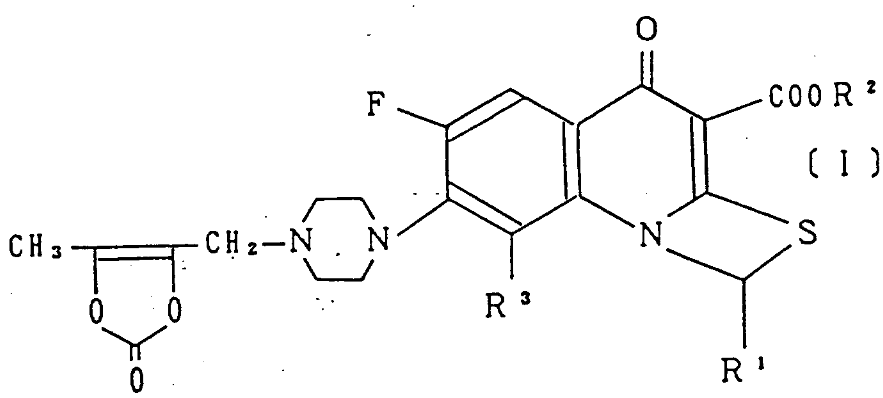

in which $R^1$ is hydrogen, straight chain or branched alkyl having 1 to 4 carbon atoms or unsubstituted or halogen-substituted phenyl; $R^2$ is hydrogen or straight chain or branched alkyl having 1 to 4 carbon atoms; and $R^3$ is hydrogen, halogen or straight chain or branched chain alkoxy having 1 to 4 carbon atoms.

(Prior Art)

Nalidixic acid, piromidic acid, pipemidic acid, enoxacin (AT-2266), ofloxacin (DL-8280), have been widely used as synthetic antibacterials for therapy of infections by gram-negative bacteria. However they are not satisfactory for therapy of infectious diseases by gram-positive bacteria and chronic infectious diseases by Pseudomonas aeruginosa which have been increasing in recent years and are hard to be cured. In order to overcome the problem, various compounds have been synthesized and many patent applications have been filed.

The present inventors have synthesized various compounds too, found quinolinecarboxylic acids having excellent antibacterial activity, and filed a patent application already (Japanese Patent Application No. 79993/1987).

Thiazetoquinolinecarboxylic acid derivatives having a general formula similar to the above-indicated formula (I) are disclosed in European Patent Application No. 249 043.

However, this reference does not mention that thiazetoquinolinecarboxylic acid derivatives having the 7-position of the quinoline moiety substituted with 4-(5-methyl-2-oxo-1,3-dioxolen-4-yl) methyl 1-piperazinyl have advantageous properties distinguishing them from the other compounds having the disclosed general formula.

European Patent Application No. 191 390 discloses quinolinecarboxylic acid derivatives having the 7-position of the quinoline moiety substituted with 4-(5-methyl-2-oxo-1,3-dioxolen-4-yl) methyl l-piperazinyl, but there is 1-cyclopropyl instead of a thiazetidine ring formed between nitrogen atom and sulfur atom in the 2-mercaptoquinolone moiety.

Copending European Patent Application No. 315 827 of the same applicants as those of the present application discloses thiazetoquinolinecarboxylic acid derivatives having a formula similar to the formula (I) of the compounds of the present invention but with the 7-position of the quinoline moiety substituted with a five to six membered cyclic amino group which may be substituted with alkyl having 1 to 4 carbon atoms, or with amino or alkyl-amino and may further contain nitrogen, oxygen or sulfur atom, instead of with 4-(5-methyl-2-oxo-1,3-dioxolen-4-yl) methyl 1-piperazinyl.

European Patent Application No. 67 411 discloses some starting compounds for the obtention of the compounds of the present invention.

(Problems to be solved by the Invention)

Though those antibacterials exhibit excellent activity, they are not still satisfactory in terms of their

bioavailability.

During the course of continued investigations to overcome such a difficulty, the present inventors have found a group of compounds exhibiting far better bioavailability than the above ones, and achieved the present invention.

Accordingly, an object of the present invention is to develop novel pharmaceuticals having far better bioavailability than the already-known synthetic antibacterials.

(Means to solve the Problems)

Compounds of the present invention are novel and have not been disclosed in the prior art literatures yet. The characteristic feature of them in terms of chemical structure is in the following two points:

1. A ring formed between nitrogen atom and sulfur atom in the 2-mercaptoquinolone skeleton is thiazetidine; and

2. 6- and 7-positions of the quinoline skeletone are substituted with fluorine and with N-(5-methyl-2-oxo-1,3-dioxolen-4-yl)methyl, respectively.

As to alkyl represented by $R^1$ and $R^2$ in the general formula (I), lower alkyl either straight chain or branched having 1 to 4 carbon atoms is preferred and is, for example, methyl, ethyl, n-propyl, isopropyl, n-butyl, isobutyl, sec-butyl, tert-butyl.

Examples of substituents for phenyl represented by $R^1$ are one to several halogens (e.g. fluorine, chlorine, bromine, iodine.) and, among those, fluorine is preferred.

As to alkoxy represented by $R^3$, lower alkoxy either straight chain or branched having 1 to 4 carbon atoms is preferred and is, for example, methoxy, ethoxy, n-propoxy, isopropoxy, n-butoxy, isobutoxy, sec-butoxy, tert-butoxy.

Examples of halogen are chlorine, bromine, iodine, fluorine, and, among those, fluorine is particularly preferred.

Examples of salts of the compound (I) of the present invention are salts with mineral acids such as hydrochloric acid, sulfuric acid, nitric acid, phosphoric acid, hydrofluoric acid, hydrobromic acid; salts with organic acids such as formic acid, acetic acid, tartaric acid, lactic acid, citric acid, fumaric acid, maleic acid, succinic acid, methanesulfonic acid, ethanesulfonic acid, benzenesulfonic acid, toluenesulfonic acid, naphthalenesulfonic acid, camphorsulfonic acid; and salts with alkali metals or alkali earth metals such as sodium, potassium, calcium.

Compounds of the present invention may, for example, be prepared in accordance with the following manner.

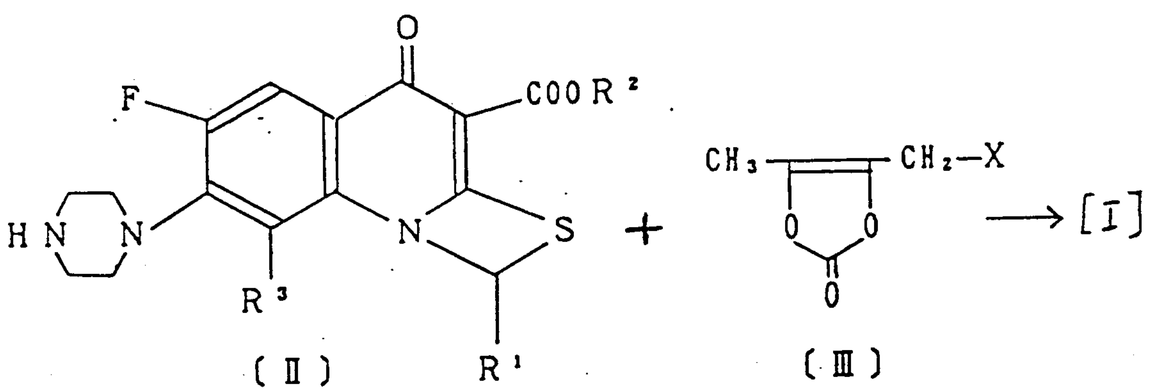

(II) (III)

wherein the meanings of $R^1$, $R^2$ and $R^3$ are the same as those defined already and X is halogen.

The compound of the formula (II) is made to react with the compound of the formula (III) in the presence or absence of a solvent which is inactive to the reaction in the presence of a base (e.g. sodium carbonate, potassium carbonate, sodium bicarbonate, potassium bicarbonate, triethylamine.) usually at -20 to +80 °C, preferably at -5 °C to room temperature, to afford (I).

Examples of the solvent used are preferably aprotic ones such as N,N-dimethylformamide, dimethyl sulfoxide, ethers such as diglyme.

The amount of (III) is preferably equimolar or an excess to one mole of (II). Reaction time may vary depending upon the type and amount of the starting materials, solvents or base, and the reaction temperature but, usually, it is from 2 to 20 hours.

When the compounds prepared in accordance with the above method is an ester ($R^2$ is alkyl), it may, if and when desired, be hydrolyzed to give the corresponding carboxylic acid ($R^2$ is hydrogen). The hydrolysis can be conducted by the use of a great excess of acid (e.g. sulfuric acid, fuming sulfuric acid, hydrochloric acid, hydrobromic acid, hydrobromic acid/acetic acid, chlorosulfonic acid, polyphosphoric acid,), preferably 10 to 20 times as much acid, as a solvent at the temperature of from room temperature to 110 ° C.

Further, the ester may be heated at 60-150 ° C, preferably at 100-110 ° C, with stirring in 10 to 100 times as much amount of alcohol corresponding to the desired ester in the presence of a catalytic amount of concentrated sulfuric acid so that the ester can be converted to desired another ester.

In the case of a carboxylic acid (i.e. $R^2$ is hydrogen), it can, if and when desired, be esterified to give desired ester (i.e. $R^2$ is alkyl). This esterification reaction can be conducted by a method known per se such as, for example, by the use of thionyl chloride with alcohol, condensing agent (e.g. dicyclocarbodiimide) with alcohol, or alkyl halide with alcoholate. Furthermore, in the case of a carboxylic acid, it can be used in a form of pharmacologically-acceptable salt such as sodium or potassium salt.

Both starting materials (II) and (III) are known and are disclosed in the specifications of Japanese Patent Application 79993/1987 and of European Patent Application No. 67 411, respectively.

The prepared compound (I) as such can be isolated and purified by a known method per se such as, for example, concentration, pH conversion, transfer to another solvent, extraction with a solvent, crystallization, recrystallization, fractional distillation, chromatography.

When the compound of the present invention is administered as a pharmaceutical drug, it is given to animals including human being in a form of a pharmaceutical composition containing 0.1 to 99.5%, preferably 0.5 to 90%, of the compound invention in a pharmaceutically-acceptable, nontoxic and inert carrier or is given to them as it is without a carrier.

Examples of the carrier applicable are one or more solid, semisolid or liquid diluent, filler or other auxiliary agent for pharmaceutical prescription. It is desired that the pharmaceutical composition is administered in a unit dose form. The pharmaceutical composition of the present invention can be administered via mouth, tissue, local place (e.g. skin) or rectum. Needless to say, the pharmaceutical form is selected suitably for each administration route. Oral administration is, for example, especially preferred.

Dose as a remedy for infectious diseases is desirably adjusted by taking the conditions of the patient (e.g. age, body weight), administration route, and type and degree of the disease into consideration but, usually, it is within a range of 50 to 1000 mg of the present invention compound/human being/day and, preferably, 100 to 330 mg. In some cases, less dose may be sufficient while in other case, more dose may be necessary. It is desired to administer dividedly, i.e. 2 or 3 times a day.

(Examples)

The present invention will be further illustrated by giving working examples showing the manufacture of the present invention compounds and by giving test examples of the present invention compounds as hereinafter.

Example 1. Ethyl 6-fluoro-1-methyl-7-[4-(5-methyl-2-oxo-1,3-dioxolen -4-yl)methyl-1-piperazinyl]-4-oxo-4H-[1,3-]thiazeto[3,2-a]quinoline-3-carboxylate.

Ethyl 6-fluoro-1-methyl-4-oxo-7-(1-piperazinyl)-4H-[1,3]thiazeto[3,2-a]quinoline-3-carboxylate (3.88 g) and 1.23 g of potassium bicarbonate were suspended in 20 ml of N,N-dimethylformamide, 2.38 g of 4-bromomethyl-5-methyl-1,3-dioxolen-2-one was dropped thereinto with ice cooling, and the mixture was stirred for 3 hours. After the reaction, the solvent was evaporated in vacuo therefrom at 50 ° C and the residue was extracted with chloroform containing a few amount of methanol. The extract was washed with water, dried, the solvent was evaporated therefrom and the residue was purified by a column chromatography (chloroform-methanol/silica gel) to give 3.32 g of desired product. M.p. 241-243 ° C (decompn.)

| Elem. Anal. for $C_{23}H_{24}FN_3O_6S$ | | | |
|---|---|---|---|
| Calcd. (%) | C : 56.43 | H : 4.94 | N : 8.58 |
| Found (%) | C : 56.13 | H : 4.99 | N : 8.26 |

I R (KBr) $\nu$ ($cm^{-1}$) : 1820, 1720 (carbonyl),

NMR ($CF_3CO_2D$)(ppm)

1.51(3H, $COOCH_2\underline{CH_3}$, t), 2.31(3H, $\underline{CH_3}$ 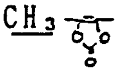, s),
2.35(3H, 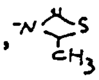, d), 3.40~4.30(8H, proton in piperazine ring, m), 4.55(2H, 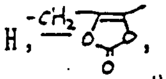, s),
4.65(2H, $COO\underline{CH_2}CH_3$, q), 6.51(1H, 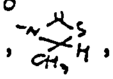, q),
7.05(1H, 8-proton, d), 8.11(1H, 5-proton, d)

Example 2. 6-fluoro-1-methyl-7- [4-(5-methyl-2-oxo-1,3-dioxolen-4-yl)methyl-1-piperazinyl]-4-oxo-4H-[1,3]thiazeto[3,2-a]quinoline-3-carboxylic acid.

6-fluoro-1-methyl-4-oxo-7-(1-piperazinyl)-4H-[1,3]-thiazeto[3,2-a]quinoline-3-carboxylic acid (2.5 g) and 1.52 g of potassium bicarbonate were suspended in 40 ml of N,N-dimethylformamide, 1.52 g of 4-bromomethyl-5-methyl-1,3-dioxolen-2-one was dropped thereinto with ice cooling, and the mixture was stirred for 3 hours. After the reaction, the mixture was evaporated in vacuo at 60 ° C, the residue was poured over into ice water, insoluble substance was collected by filtration, washed with water, air-dried, the resulting crude crystals were recrystallized from chloroform-methanol (10:1), and 2.05 g of the desired product was obtained. M.p. 138-140 ° C (decompn.).

| Elem. Anal. for $C_{21}H_{20}FN_3O_6S \cdot 1\,^2/_3H_2O$ | | | |
|---|---|---|---|
| Calcd. (%) | C : 51.32 | H : 4.79 | N : 8.55 |
| Found (%) | C : 51.39 | H : 4.94 | N : 8.30 |

I R (KBr) ν ($cm^{-1}$) : 1815, 1700

NMR ($CF_3CO_2D$)(ppm)

2.31(3H, 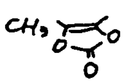, t), 2.35(3H, 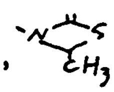, d),
3.40~4.30(8H, proton in piperazine ring, m),
4.55(2H, 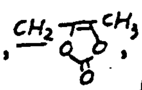, s),
6.55(1H, 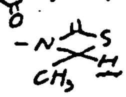, q), 7.05(1H, 8-proton, d), 8.15(1H, 5-proton, d)

Example 3. 6-Fluoro-1-methyl-7-[4-(5-methyl-2-oxo-1,3-dioxolen-4-yl)methyl-1-piperazinyl]-4-oxo-4H-[1,3]thiazeto[3,2-a]quinoline-3-carboxylic acid hydrochloride.

The compound (500 mg) prepared in Example 2 was dissolved in chloroform and 0.5 ml of 33% methanolic hydrochloric acid was added thereto. The crystals separated out therefrom were collected by filtration, washed with small amount of methanol and then with ether, and dried in vacuo to give 470 mg of desired product, m.p. 234-237 ° C (decompn.).

| Elem. Anal. for $C_{21}H_{20}FN_3O_6S.HCl.2H_2O$ | | | |
|---|---|---|---|
| Calcd (%)<br>Found (%) | C: 47.24<br>C: 47.54 | H: 4.72<br>H: 4.59 | N: 7.87<br>N: 7.77 |

Example 4. 6-Fluoro-1-methyl-7-[4-(5-methyl-2-oxo1,3-dioxolen-4-yl)methyl-1-piperazinyl]-4-oxo-4H-[1,3]thiazeto[3,2-a]quinoline-3-carboxylic acid methanesulfonate.

Similarly prepared was the title product by the same manner as in Example 3. M.P. 230-233 °C (decompn.).

| Elem. Anal. for $C_{21}H_{20}FN_3O_6S.CH_3SO_3H.1\frac{1}{2}H_2O$. | | | |
|---|---|---|---|
| Calcd (%):<br>Found (%): | C: 45.20<br>C: 45.14 | H: 4.66<br>H: 4.50 | N: 7.19<br>N: 7.01 |

Example 5. 6-Fluoro-7-[4-(5-methyl-2-oxo-1,3-dioxolen-4-yl)methyl1-piperazinyl]-4-oxo-1-phenyl-4H-[1,3]thiazeto[3,2-a]quinoline-3-carboxylic acid.

6-Fluoro-4-oxo-1-phenyl-7-(1-piperazinyl)-4H-[1,3]thiazeto[3,2-a]quinoline-3-carboxylic acid (3.0 g) and 0.88 g of potassium bicarbonate were suspended in 50 ml of N,N-dimethylformamide, 1.69 g of 4-bromomethyl-5-methyl1,3-dioxolen-2-one was dropped thereinto with ice cooling, and the mixture was stirred for 3 hours. After the reaction, the mixture was poured into ice water, the crystals separated out therefrom was collected by filtration, washed with water, dried in vacuo, and the resulting crude crystals were recrystallized from chloroform-ethanol to give 1.3 g of desired product, m.p. 201-202 °C (decompn.).

| Elem. Anal. for $C_{26}H_{22}FN_3O_6S \cdot \frac{3}{4}H_2O$ | | | |
|---|---|---|---|
| Calcd. (%)<br>Found (%) | C : 58.15<br>C : 58.10 | H : 4.41<br>H : 4.31 | N : 7.82<br>N : 7.80 |

I R (KBr) $\nu$ ($cm^{-1}$) : 1810, 1710

NMR ($CF_3CO_2D$)(ppm)

2.25(3H, 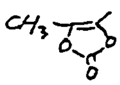 , s),

3.00~4.30(8H, proton in piperazine ring, m),

4.42(2H, 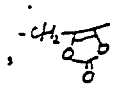, s),

6.43(1H, 8-proton, d), 7.25(1H, 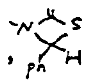, s),

7.53(5H, ph, s), 8.08(1H, 5-proton, d)

Example 6. Ethyl 6-fluoro-7-[4-(5-methyl-2-oxo-1,3-dioxolen-4-yl)methyl-1-piperazinyl]-4-oxo-1-phenyl-4H-[1,3]-thiazeto[3,2-a]quinoline-3-carboxylate.

Ethyl 6-fluoro-4-oxo-1-phenyl-7-(1-piperazinyl)-4H-[1,3]thiazeto[3,2-a]quinoline-3-carboxylate (3.0 g) and 0.82 g of potasium bicarbonate were suspended in 50 ml of N,N-dimethylformamide, 1.58 g of 4-bromomethyl-5-methyl-1,3-dioxolen-2-one was dropped thereinto with ice cooling, and the mixture was stirred for 3 hours. After the reaction, the mixture was poured over into ice water and the crystals separated out therefrom were collected by filtration. They were then dissolved in chloroform, the solution was washed with water, and evaporated/dried in vacuo. The resulting oily residue was purified by a column chromatography (silica gel/methanol-chloroform [1:50]) to give 2.47 g of desired product, mp. 223-226 °C (decompn.).

| Elem. Anal. for $C_{28}H_{26}FN_3O_6S \cdot \frac{1}{2}H_2O$ | | | |
|---|---|---|---|
| Calcd. (%)<br>Found (%) | C : 59.99<br>C : 60.10 | H : 4.85<br>H : 5.04 | N : 7.50<br>N : 7.34 |

NMR ($CF_3CO_2D$) (ppm)

1.54 (3H, $COOCH_2C\underline{H_3}$, t), 2.25 (3H, 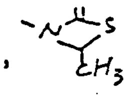, d),
2.28 (3H, $-CH_3$, s),
3.10~4.20 (8H, proton in piperazine ring, m),
4.47 (2H, 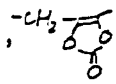, s), 4.65 (2H, $COO\underline{CH_2}-CH_3$, q)
6.45 (1H, 8-proton, d), 7.28 (1H, 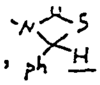, s),
7.55 (5H, ph., s), 8.10 (1H, 5-proton, d)

Similarly prepared were the following compounds.

Example 7. 6,8-difluoro-7-[4-(5-methyl-2-oxo-1,3-dioxolen-4-yl)methyl-1-piperazinyl]-4-oxo-4H-[1,3]-thiazeto[3,2-a]quinoline-3-carboxylic acid.

M.p. 255 °C (decompn)

| Elem Anal. for $C_{20}H_{17}F_2N_3O_6S$ | | | |
|---|---|---|---|
| Calcd (%)<br>Found (%) | C: 51.61<br>C: 51.92 | H: 3.68<br>H: 3.85 | N: 9.03<br>N: 8.57 |

Mass analysis ($C_{20}H_{17}F_2N_3O_6S$), $M^+$: 465.

Example 8. 6,8-difluoro-1-methyl-7-[4-(5-methyl-2-oxo-1,3-dioxolen-4-yl)methyl-1-piperazinyl]-4-oxo-4H-[1,3]thiazeto[3,2-a]quinoline-3-carboxylic acid.

M.p. 168-171 °C

| Elem. Anal. for $C_{21}H_{19}F_2N_3O_6S.\frac{1}{2}H_2O$ | | | |
|---|---|---|---|
| Calcd (%)<br>Found (%) | C: 51.63<br>C: 51.47 | H: 4.13<br>H: 3.92 | N: 8.60<br>N: 8.46 |

Mass analysis ($C_{21}H_{19}F_2N_3O_6S$), $M^+$: 479.

Example 9. 6,8-difluoro-7-[4-(5-methyl-2-oxo-1,3-dioxolen-4-yl)methyl-1-piperazinyl]-4-oxo-1-phenyl-4H-[1,3]thiazeto[3,2-a]quinoline-3-carboxylic acid.

M.p. 160-161 °C (decompn)

| Elem. Anal. for $C_{26}H_{21}F_2N_3O_6S$ | | | |
|---|---|---|---|
| Calcd (%)<br>Found (%) | C: 57.61<br>C: 57.26 | H: 3.91<br>H: 3.97 | N: 7.76<br>N: 7.64 |

Mass analysis ($C_{26}H_{21}F_2N_3O_6S$), $M^+$: 541.

Example 10. 6,8-difluoro-1-(4-fluorophenyl)-7-[4-(5-methyl-2-oxo-1,3-dioxolen-4-yl)methyl-1-piperazinyl]-4-oxo-4H-[1,3]thiazeto[3,2-a]quinoline-3-carboxylic acid.

Mass analysis ($C_{26}H_{20}F_3N_3O_6S$), $M^+$: 559.

Example 11. 6,8-difluoro-1-(2,4-difluorophenyl)-7-[4-(5-methyl-2-oxo-1,3-dioxolen-4-yl)methyl-1-piperazinyl]-4-oxo-4H-[1,3]thiazeto[3,2-a]quinoline-3-carboxylic acid.

Mass analysis ($C_{26}H_{19}F_4N_3O_6S$), $M^+$: 577.

Example 12. 6,8-difluoro-1-(3,4-difluorophenyl)-7-[4-(5-methyl-2-oxo-1,3-dioxolen-4-yl)methyl-1-piperazinyl]-4-oxo-4H-[1,3]thiazeto[3,2-a]quinoline-3-carboxylic acid.

Mass analysis ($C_{26}H_{19}F_4N_3O_6S$), $M^+$: 577.

Example 13. 6-fluoro-8-methoxy-7-[4-(5-methyl-2-oxo-1,3-dioxolen-4-yl)methyl-1-piperazinyl]-4-oxo-4H-[1,3]thiazeto[3,2-a]quinoline-3-carboxylic acid.

Mass analysis ($C_{21}H_{20}FN_3O_7S$), $M^+$: 515.

Example 14. 6-fluoro-8-methoxy-1-methyl-7-[4-(5-methyl-2-oxo-1,3-dioxolen-4-yl)methyl-1-piperazinyl]-4-oxo-4H-[1,3]thiazeto[3,2-a]quinoline-3-carboxylic acid.

M.p. 158-159 °C

| Elem. Anal. for $C_{22}H_{22}FN_3O_7S$ | | | |
|---|---|---|---|
| Calcd (%) | C: 53.76 | H: 4.51 | N: 8.55 |
| Found (%) | C: 53.47 | H: 4.64 | N: 8.69 |

Mass analysis ($C_{22}H_{22}FN_3O_7S$), $M^+$: 529.

Example 15. 6-fluoro-8-methoxy-7-[4-(5-methyl-2-oxo-1,3-dioxolen-4-yl)methyl-1-piperazinyl]-4-oxo-1-phenyl-4H-[1,3]thiazeto[3,2-a]quinoline-3-carboxylic acid.

Mass analysis ($C_{27}H_{24}FN_3O_7S$), $M^+$: 591.

Test Example.

As hereunder, result of pharmacological test showing the usefulness of the representative compounds of the present invention is given.

2. Therapeutic effect to infection in mice.

Test method: E. coli KC-14 and P. aeruginosa E-2 were suspended in 5% mucin and 0.5 ml of the suspension was injected intraperitoneally to ddY strain male mice (body weight: Ca. 20 g; four weeks age; 10 mice per group). The amount of the bacteria inoculated was 5.1 x $10^4$ CFU/mouse for E. coli and 7.5 x $10^4$ CFU/mouse for P. aeruginosa. Drug was given orally once after 2 hours of inoculation and, out of the survival rate after one week, $ED_{50}$ was calculated by a Probit method. As to a comparison/control, ofloxacin was used. The result is given in Table 2.

Table 2

| Compound Tested (Example Number) | $ED_{50}$ (mg / mouse) | |
|---|---|---|
| | E. coli | P. aeruginosa |
| 1 | - | 0.354 |
| 2 | 0.0078 | 0.154 |
| 3 | 0.0078 | 0.125 |
| 4 | 0.0078 | 0.125 |
| 8 | 0.0078 | 0.0152 |
| 14 | 0.125 | 0.427 |
| Ofloxacin | 0.011 | 0.692 |

It is apparent that the present invention compounds exhibit strong therapeutic effect to infectious diseases to mice.

(Effect)

It is obvious from the above facts and results that the compounds of the present invention are effective at far less doses than the conventional antibacterial agents not only to P. aeruginosa but also both gram-positive and negative bacteria and they exhibit wide antibacterial spectrum.

Moreover, their absorption after oral administration is better than the conventional drugs whereupon they are converted to active form promptly showing good therapeutic effect.

Furthermore the compounds of the present invention are very little toxic and, accordingly, they can be applied, with high safety, as therapeutic agents to systemic infectious diseases and local ones such as infectious diseases in urinary gall tracts of mammals including human being.

## Claims

**Claim for the following Contracting States : AT, BE, CH, DE, FR, GB, IT, LI, NL, SE**

**1.** Quinolinecarboxylic acid derivative represented by the following general formula (I) and physiologically-acceptable salt thereof:

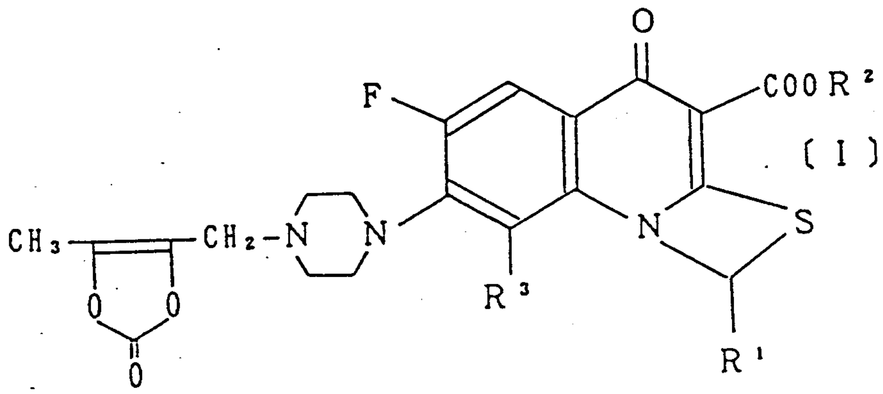

in which $R^1$ is hydrogen, straight chain or branched alkyl having 1 to 4 carbon atoms or unsubstituted or halogen-substituted phenyl; $R^2$ is hydrogen or straight chain or branched alkyl having 1 to 4 carbon atoms; and $R^3$ is hydrogen, halogen or straight chain or branched chain alkoxy having 1 to 4 carbon atoms.

**Claim for the following Contracting State : ES**

**1.** Process for preparation of quinolinecarboxylic acid derivatives represented by the following general formula (I)

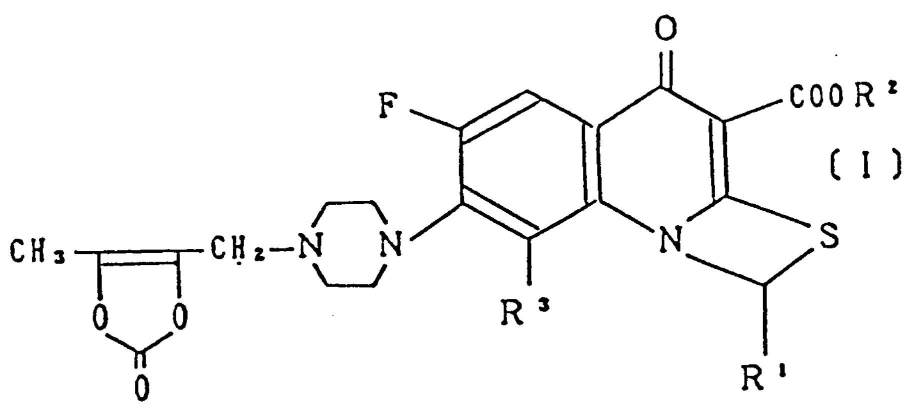

in which $R^1$ is hydrogen, straight chain or branched alkyl having 1 to 4 carbon atoms or unsubstituted or halogen-substituted phenyl; $R^2$ is hydrogen or straight chain or branched alkyl having 1 to 4 carbon atoms; and $R^3$ is hydrogen, halogen or straight chain or branched chain alkoxy having 1 to 4 carbon atoms, characterized in that a compound of the formula (II)

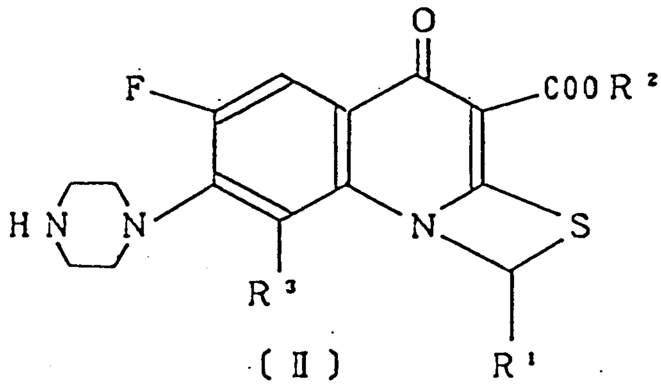

(II)

is made to react with a compound of the formula (III)

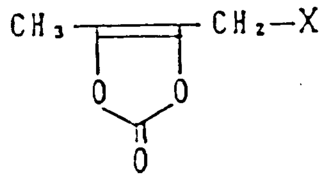

(III)

in the presence of a base, to afford a compound of the formula (I).

**Revendications**

**Revendication pour les Etats contractants suivants : AT, BE, CH, DE, FR, GB, IT, LI, NL, SE**

1. Dérivé de l'acide quinoléine carboxylique représenté par la formule générale (I) suivante et ses sels physiologiquement acceptables :

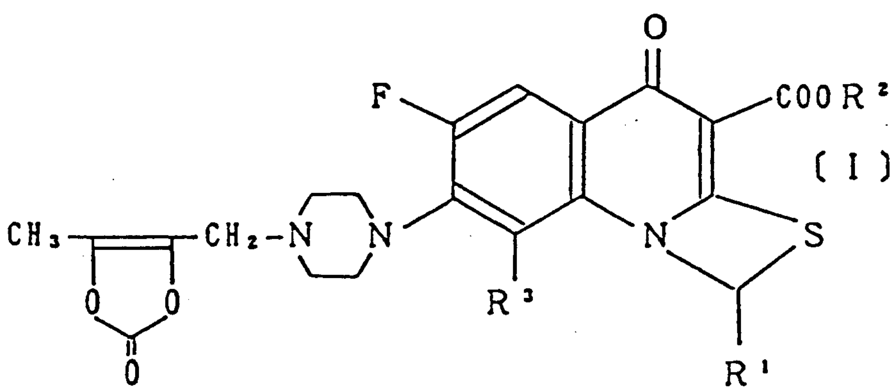

(I)

dans laquelle $R^1$ est un atome d'hydrogène, un groupe alkyle à chaîne linéaire ou ramifiée en $C_1$ à $C_4$ ou un groupe phényle non substitué ou substitué par un halogène; $R^2$ est un atome d'hydrogène ou un groupe alkyle à chaîne linéaire ou ramifiée en $C_1$ à $C_4$; et $R^3$ est un atome d'hydrogène, un atome d'halogène ou un groupe alcoxy à chaîne linéaire ou ramifiée en $C_1$ à $C_4$.

**Revendication pour l'Etat contractant suivant : ES**

1. Procédé de préparation de dérivés de l'acide quinoléine carboxylique représentés par la formule générale (I) suivante:

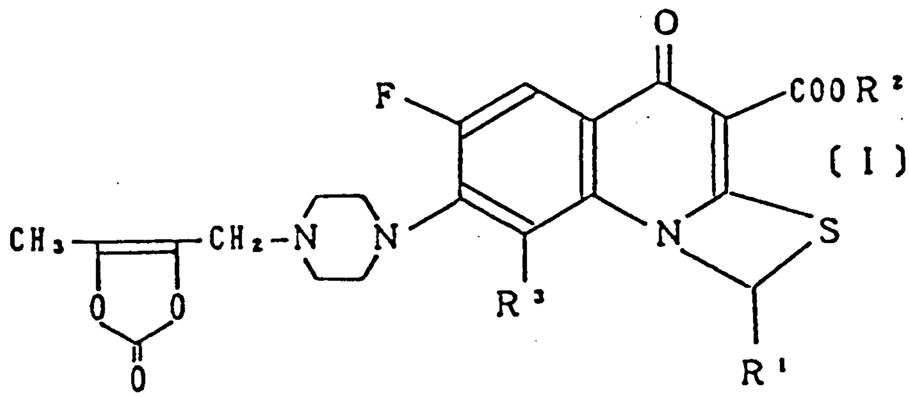

dans laquelle $R^1$ est un atome d'hydrogène, un groupe alkyle à chaîne linéaire ou ramifiée en $C_1$ à $C_4$ ou un groupe phényle non substitué ou substitué par un halogène; $R^2$ est un atome d'hydrogène ou un groupe alkyle à chaîne linéaire ou ramifiée en $C_1$ à $C_4$; et $R^3$ est un atome d'hydrogène, un atome d'halogène ou un groupe alcoxy à chaîne linéaire ou ramifiée en $C_1$ à $C_4$, caractérisé en ce qu l'on fait réagir un composé de formule (II)

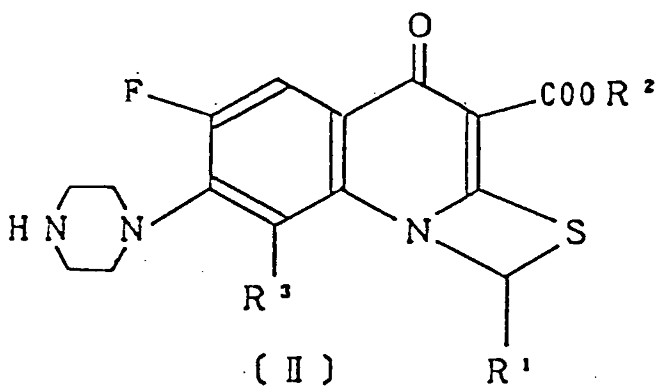

(II)

avec un composé de formule (III)

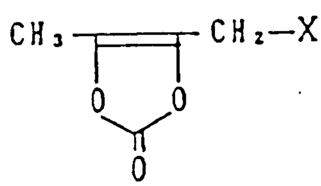

(III)

en présence d'une base, de façon à obtenir un composé de formule (I).

**Patentansprüche**

**Patentanspruch für folgende Vertragsstaaten : AT, BE, CH, DE, FR, GB, IT, LI, NL, SE**

1. Chinolincarbonsäurederivat der nachfolgenden allgemeinen Formel (I) und dessen physiologisch annehmbare Salze:

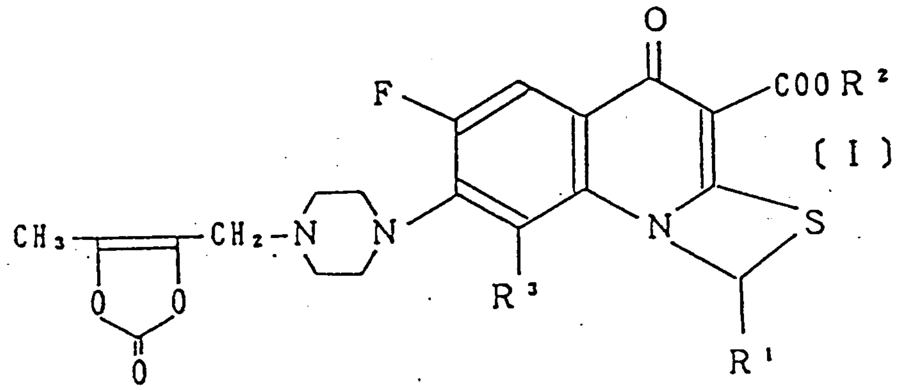

worin $R^1$ Wasserstoff, geradkettiges oder verzweigtes Alkyl mit 1 bis 4 Kohlenstoffatomen oder unsubstituiertes oder halogensubstituiertes Phenyl ist; $R^2$ Wasserstoff oder geradkettiges oder verzweigtes Alkyl mit 1 bis 4 Kohlenstoffatomen ist; und $R^3$ Wasserstoff, Halogen oder geradkettiges oder verzweigtes Alkoxy mit 1 bis 4 Kohlenstoffatomen ist.

**Patentanspruch für folgenden Vertragsstaat : ES**

**1.** Verfahren zur Herstellung von Chinolincarbonsäurederivaten der nachfolgenden allgemeinen Formel (I):

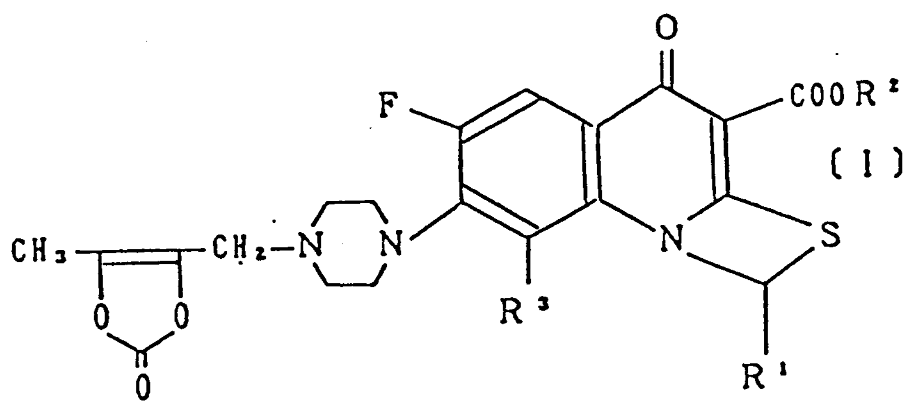

worin $R^1$ Wasserstoff, geradkettiges oder verzweigtes Alkyl mit 1 bis 4 Kohlenstoffatomen oder unsubstituiertes oder halogen-substituiertes Phenyl ist; $R^2$ Wasserstoff oder geradkettiges oder verzweigtes Alkyl mit 1 bis 4 Kohlenstoffatomen ist; und $R^3$ Wasserstoff, Halogen oder geradkettiges oder verzweigtes Alkoxy mit 1 bis 4 Kohlenstoffatomen ist, dadurch gekennzeichnet, dass eine Verbindung nach Formel (II):

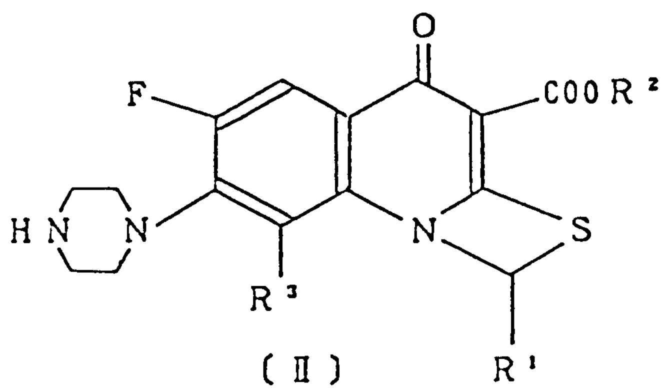

mit einer Verbindung nach Formel (III):

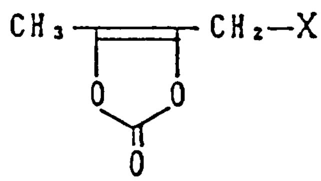

(III)

im Beisein einer Base zur Reaktion gebracht wird, um hierdurch eine Verbindung nach Formel (I) zu ergeben.